# EUROPEAN PATENT SPECIFICATION

(11) **EP 4 489 707 B1**
(45) Date of publication and mention of the grant of the patent: **14.01.2026**
(21) Application number: 22713573.8
(22) Date of filing: 07.03.2022
(51) Int. Cl.: A61F 13/47, A61F 13/511, A61F 13/84

(54) **SANITARY PAD COMPRISING A WATER-SOLUBLE FORMULATION**
HYGIENEVORLAGE MIT EINER WASSERLÖSLICHEN FORMULIERUNG
TAMPON HYGIÉNIQUE COMPRENANT UNE FORMULATION SOLUBLE DANS L'EAU

(43) Date of publication of application: 15.01.2025
(73) Proprietor: Essity Hygiene and Health Aktiebolag, 405 03 Göteborg (SE)
(72) Inventor: ALENIUS, Karin, 405 03 Göteborg (SE); ZOREVAND, Katarina, 405 03 Göteborg (SE); HAGBERG, Daniel, 429 33 Kullavik (SE); RYTTSÉN, Frida, 405 03 Göteborg (SE)
(74) Representative: Hoffmann Eitle
(86) International application number: PCT/EP2022/055683
(87) International publication number: WO 2023/169649

(56) References cited:
- WO-A1-2021/115607
- US-A1- 2003 114 812
- US-A1- 2019 001 017
- US-A1- 2021 220 191

## Description

### TECHNICAL FIELD

The disclosure pertains to a sanitary pad, such as a sanitary pad, a panty liner, a diaper or an incontinence pad, comprising a topsheet and a backsheet, the topsheet comprising a water-soluble formulation applied in a coated zone.

### BACKGROUND

Sanitary pads of the kind to which this disclosure relates are worn against the skin and comprise a topsheet and a backsheet layer. All uses of products which are applied in direct contact with the skin may lead to unwanted side-effects. These may occur as a result of occlusion, moisture, mechanical, microbial, and enzymatic factors which all, to different degrees, interact and amplify the influence of each other and may cause different forms of skin irritation for users of said articles.

During frequent insults of bodily fluids and frequent use of disposable sanitary pads, the skin can become irritated, appear red, and be sore to the touch. Creams, formulations, or ointments can be used to provide an artificial hydrophobic barrier on the skin. However, the use of such hydrophobic compositions has a negative impact on the absorbency performance of the articles. To avoid this side effect, hydrophobic compositions are often applied in relatively low amounts and in different patterns on the topsheet, such as in elongated stripes extending in parallel over the topsheet.

Thinner and smaller size sanitary pads, such as panty liners, may be used more frequently, sometimes daily, which may also lead to occlusion and an increased mechanical impact on the skin. Thinner and smaller sized sanitary pads may also have more flexible transverse rear and front edges and side edges which are located mainly in the proximity of the crotch area. This may result in a higher degree of undesired folding, bunching, or twisting of the edges of such articles which may create discomfort and impaired product performance. Furthermore, smaller sanitary pads having a relatively two-dimensional shape, compared to thicker more three-dimensional sanitary pads, have a higher tendency to move in relation to the skin of the user, resulting in a higher risk of friction against the skin compared to a product including for example a hump in the crotch portion and which is shaped to fit more closely to the anatomy of the user.

Separate application of creams, formulations and ointments to the skin is often messy and inconvenient. Often, these products are not used prophylactically and are only used when signs of skin irritation, such as diaper rash, are visible.

In view of the above, there is a desire to provide a comfortable sanitary pad which prevent skin irritations and provide skin benefits while maintaining satisfying performance properties.

US 2019/001017 A1 relates to a method of applying a skin beneficial agent to an absorbent article including a topsheet layer having a body facing surface and a garment facing surface, the article having a longitudinal front portion, a longitudinal back portion and a crotch portion located between the front and the back portion. The method includes at least the step of printing, by means of an in-line synchronized print technique, a water based ink composition including a binder and a microencapsulated skin beneficial agent on the article.

US 2003/114812 A1 states a feminine care absorbent article including an outer cover and an absorbent structure in superposed relation to the outer cover and defining a bodyfacing surface. A lotion formulation is deposited on the bodyfacing surface in discrete locations targeted for specific regions of the wearer's body.

### SUMMARY OF THE INVENTION

One or more of the above objects may be achieved with a sanitary pad in accordance with claim 1. Further embodiments are set out in the dependent claims, in the following description and in the drawings.

A sanitary pad, such as a pantyliner, as disclosed herein comprises a fluid permeable topsheet and a backsheet. The sanitary pad extending in a longitudinal direction and a transverse direction and having first and second longitudinal side edges and a front and a rear edge. The sanitary pad has a length extending in the longitudinal direction, as measured along a longitudinal centreline of the sanitary pad and a maximum width, as measured in the transverse direction. The sanitary pad is made up of a front third, a rear third and a central third arranged between the front and the rear third, as seen in the longitudinal direction. The topsheet is provided with a water-soluble formulation applied in a coated zone arranged along the rear edge and the first and the second longitudinal side edges in the rear third of the sanitary pad and forming a boundary in the rear third delimiting an inner rear zone comprising or consisting of a coated free topsheet area. The inner rear zone extends along the longitudinal centreline in the rear third of the sanitary pad. The coated zone is arranged at a distance from the rear edge and from the first and second longitudinal side edges and covers at least from 25%, 35% or at least from 50% of a total surface area of the topsheet in the rear third of the sanitary pad.

Optionally, the coated zone covers within the range of from 25% to 80% of the total surface area of the topsheet in the rear third of the sanitary pad. Optionally the coated zone covers from 35% to 75% of the total surface area of the topsheet in the rear third of the sanitary pad.

The term "sanitary pad" refers to a product that is placed against the skin of the wearer in the wearer's crotch to absorb and contain body exudates, like discharges, urine and menstrual fluid. The disclosure mainly refers to disposable sanitary pads, which means articles that are not intended to be laundered or otherwise restored or reused as a sanitary pad. Examples of disposable sanitary pads include feminine hygiene products such as sanitary napkins, panty liners and incontinence pads, and the like.

During use, sanitary pads remain in close contact with the skin of the user. A moist environment between the topsheet and the skin, resulting from frequent insults during use of the sanitary pads, may increase the friction and lead to skin irritations and a deteriorated skin barrier.

The present disclosure is based on the finding of the present inventors that the area of the sanitary pad which is in closest contact with the skin of the user is the area arranged along the rear edge and the first and the second longitudinal side edges in the rear third of the sanitary pad and not the central area. Furthermore, it has also been found that the area in closest contact with the skin does not include the edges as such. There is thus a benefit of providing this area with a friction reducing and skin beneficial water-soluble formulation while the inner rear zone comprises or consists of a coated free topsheet area. The fact that the coated zone is provided at a distance from the rear edge and from the first and second longitudinal side edges prevents soiling of the undergarment due to folded edges. This is particularly relevant since the present disclosure pertains to a sanitary pad with the edges located closer to the crotch area compared to for example a diaper.

The distance from the coated zone to each of the rear edge, the first and the second longitudinal edges may be 1 mm or more, optionally within the range of from 1 mm to 18 mm, optionally within the range of from 1 mm to 15 mm, such as within the range of from 1 to 10 mm. The fact that the coated zone is arranged at a distance from the edge prevents soiling of the formulation on the undergarment if the edges become folded during use.

The sanitary pad may comprise an absorbent core arranged between the topsheet and the backsheet. The sanitary pad may comprise an absorbent core free area at the front, rear and longitudinal edges where the topsheet and the backsheet are connected to each other directly or via an acquisition layer.

The absorbent core may have a generally uniform no-load thickness over the total absorbent core. The absorbent core may constitute of one absorbent layer, thus having a uniform no-load thickness.

Alternatively, the absorbent core may comprise two or more absorbent layers, and wherein each of the layers of the absorbent core may has the same extension, as seen in the longitudinal direction and the transverse direction. Since the outer edges of each of the absorbent layers thus coincides, as seen from the user-facing side of the pad, the thickness of the absorbent core is uniform.

The coated zone may be arranged along the front edge, the first and the second longitudinal side edges in the front third of the sanitary pad, the coated zone being arranged at a distance from the front edge, the first and second longitudinal side edges in the front third of the sanitary pad. Such sanitary pad provides skin benefits both in the regions rear and front region of the user.

The coated zone may form a boundary in the front third delimiting and the inner front zone comprising or consisting of a coated free topsheet area, the inner front zone may extend along the longitudinal centreline. The coated zone in the front third covers at least 25% or at least 35%, or at least 50% of the total surface area of the topsheet in the front third of the sanitary pad.

Optionally, the coated zone in the front third of the sanitary pad covers within the range from 25% to 80% of the total surface area of the topsheet in the front third of the sanitary pad. Optionally, the coated zone in the front third of the sanitary pad covers within the range from 35% to 70% of the total surface area of the topsheet in the front third of the sanitary pad.

The fact that the coated zone is provided at a distance from the rear edge and from the first and second longitudinal side edges prevents soiling of the undergarment due to bunching, twisting and folding of the edges.

The distance from the coated zone to each of the front edge, the first and the second longitudinal edges may be 1 mm or more, optionally within the range of from 1 mm to 18 mm, or 1 mm to 15 mm.

The coated zone may be arranged along the first and the second longitudinal side edges in the central third of the sanitary pad, the coated zone being arranged at a distance from the first and second longitudinal side edges in the central third of the sanitary pad and forms a boundary in the central third delimiting an inner central zone comprising or consisting of a coated-free topsheet area, the inner central coated free zone extending along the longitudinal centreline, optionally the inner central coated free zone covers at least 40% of the total area of the topsheet in the central third of the sanitary pad. This provides improved skin benefits in the crotch area of the user while preserving the absorbency capabilities of the sanitary pad in the central crotch region. According to this embodiment, the coated zone is arranged to provide an outer border in the central third of the sanitary pad. delimiting an inner central coated free zone. The coated zone may cover at least 25%, or at least 35% or at least 50%, of a total surface area of the topsheet in the central third of the sanitary article. Optionally the coated zone covers within the range of from 25% to 80%, such as within the range of from 35 to 75% of the total surface area of the topsheet in the central third of the sanitary article.

The distance from the coated zone to each of the first and the second longitudinal edges may be 1 mm or more, optionally within the range of from 1 mm to 18 mm.

The length of the sanitary pad may be within the range of from 100 to 300 mm, or 100 to 260 mm. Such shorter sanitary pads may have a more prominent risk of folding of the edges of the sanitary pad since the front and rear edges are located closer to the crotch region of the user which region may have a higher risk of cause bunching, twisting or folding of the sanitary pad edges.

The maximum width of the sanitary pad may be within the range of from 35 mm to 110 mm. Such narrower sanitary pads may have a more prominent risk of folding of the edges of the sanitary pad since the longitudinal edges are located closer to the crotch region of the user which region may have a higher risk of cause folding of the sanitary pad edges. If there is formulation provided on the edges, there is a risk of soiling of the undergarment. If the pad is provided with wings, i e two wing-shaped fastening elements extending outwardly from the main body to allow fastening of the pad to the undergarment, the width of the wings are excluded when measuring the maximum width of the sanitary pad.

The amount of formulation in the coated zone may be within the range of from 0.2 gsm to 12 gsm, or 0.5 gsm to 7 gsm or 0.5 gsm to 3 gsm. In a sanitary pad when the coated zone is provided according to the present disclosure it has been found by the present inventors that such amounts of formulation are particularly suited for the intended purpose.

The water-soluble formulation may comprise for example allantoin and/or betaine. The water-soluble formulation may comprise a water miscible vehicle, such as glycerine, propylene glycol and/or PEG. The water-soluble formulation may be free from oil and from emulsifier, the water-soluble formulation could alternatively be free from non-encapsulated oil and/or emulsifiers.

The water-soluble formulation may be applied within the coated zone in an amount with the range of from 0.2 to 12 gsm, optionally in an amount within the range of from 0.5 to 7 gsm, or in an amount within the range of from 0.5 to 3.0 gsm. The aqueous formulation may be added to the topsheet prior to assembly of the sanitary article or to the topsheet of the assembled sanitary article, prior to or after cutting out the individual sanitary article. The topsheet may be dried after the aqueous formulation is applied and the amount of the coating is measured after drying the topsheet, so the amount of coating defined in this application is the amount of coating on the article during use of the article. After the drying step, the coating may be a dry coating. The formulation may be provided continuously in the coated zone. The formulation may alternatively be discontinuously applied, such as for example in a dotted or striped pattern.

The coated free zone, being defined by an inner contour of the coated zone, may form a head region, a body region and a neck region, the neck region forming a transitional region between the head region and the body region, the head region being positioned in the front half of the sanitary pad and the body region being positioned in the front half and in the rear half of the sanitary pad, wherein a transverse width of the coated free zone at the neck region is smaller than a maximum transverse width of the head region. This provides an increased coated zone in the groin area which may be an area in close contact with the sanitary pad and therefore particularly exposed to friction and thus benefitting from the skin beneficial action of the water-soluble formulation.

The topsheet may comprise a second side being an opposite side to the first side, the second side of the topsheet being printed in a printed zone and the coated zone on the first side of the topsheet may partly or completely overlap with the printed zone, as seen in a depth direction perpendicular to the longitudinal direction. This may provide a visual cue to the user of where the water-soluble coating s located which may provide the user with indication of how to place the pad, but also provide the user with signal of the presence of the skin benefit formulation The print may have different colors or different shades of the same color. It is also possible to have one printed zone to visualizing the presence of the skin benefit coating and another printed zone visualizing for example the central wetting area.

### BRIEF DESCRIPTION OF THE DRAWINGS

The present invention will be further explained hereinafter by means of non-limiting examples and with reference to the appended drawings wherein:
- Fig. 1: illustrates a perspective view of a sanitary pad according to the present disclosure;
- Fig. 2: is a plan view of the sanitary pad from Fig. 1;
- Fig. 3: is an exploded view of the sanitary pad from Fig. 1; and
- Fig. 4: is a top plan view of a sanitary pad according to the present disclosure.

### DETAILED DESCRIPTION

It is to be understood that the drawings are schematic and that individual components, such as layers of material are not necessarily drawn to scale. The sanitary pad shown in the figures are provided as an example only and should not be considered limiting to the invention as disclosed herein.

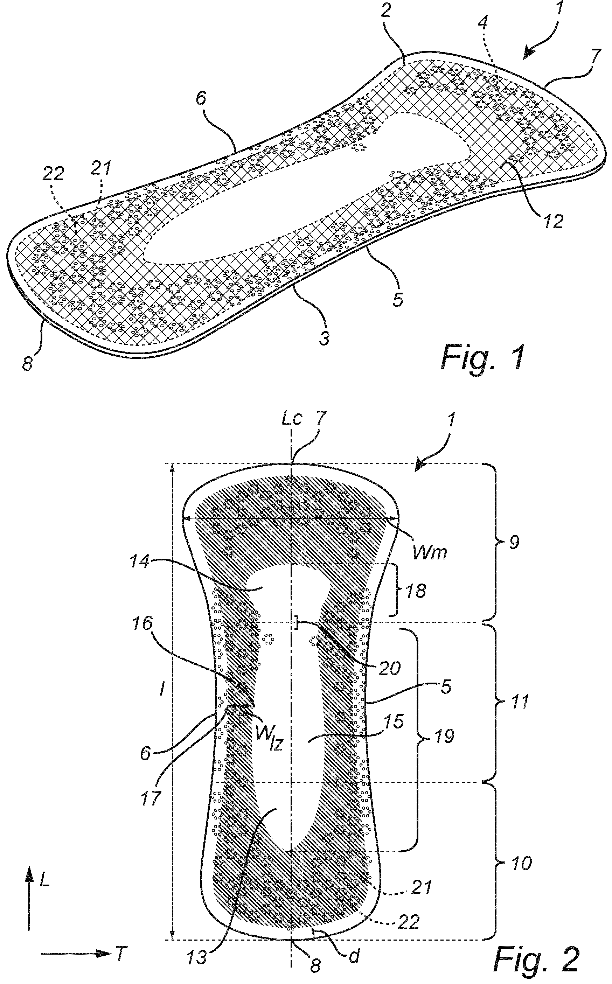

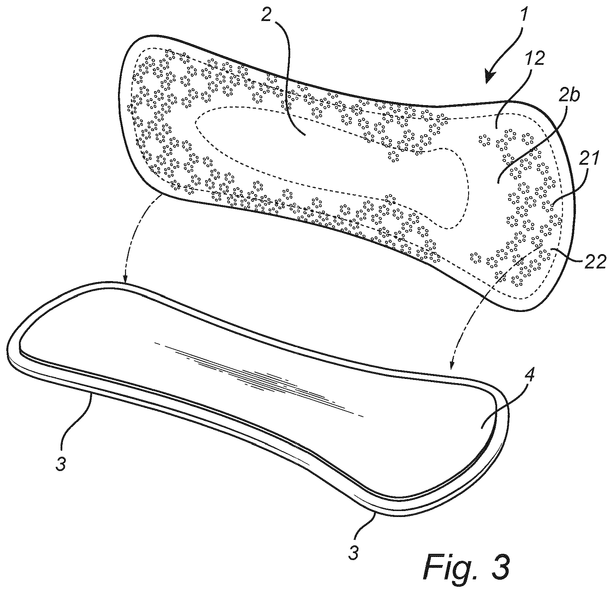

Figs. 1 -3 show a sanitary pad 1 according to the present disclosure. The sanitary pad 1 comprises a topsheet 2 and a backsheet 3. An absorbent core 4 is arranged between the topsheet 2 and the backsheet 3. The topsheet 2 has a user-facing side 2a and an opposite side 2b facing away from the user.

The sanitary pad may be any one of feminine hygiene products such as sanitary napkins, panty liners, incontinence pads, diapers, and the like. The diapers can be open diapers and pant diapers.

The sanitary pad 1 extends in a longitudinal direction L and a transverse direction T and has a first longitudinal side edge 5, a second longitudinal side edges 6, a front edge 7 and a rear edge 8. The sanitary pad 1 has a length I extending in the longitudinal direction L and being measured along a longitudinal centreline Lc of the sanitary pad 1 and a maximum width Wm, as measured in the transverse direction T. The sanitary pad 1 is made up of a front third 9, a rear third 10 and a central third 11 arranged between the front third 9 and the rear third 10, as seen in the longitudinal direction L. The topsheet 2 is provided with a water-soluble formulation, the water-soluble formulation being applied in a coated zone 12 arranged along the rear edge 8 and the first and the second longitudinal side edges 5,6 in the rear third 10 of the sanitary pad 1. The coated zone 12 forms a boundary in the rear third delimiting an inner rear zone 13 comprising or consisting of a coated free topsheet area. The inner rear zone 13 extends along the longitudinal centreline Lc The coated zone 12 is arranged at a distance d from the rear edge 8 and from the first and second longitudinal side edges 5,6. The coated zone covers within the range of from 25% to 80% of the total surface area of the topsheet in the rear third of the sanitary pad. Optionally the coated zone covers from 35% to 75% of the total surface area of the topsheet in the rear third of the sanitary pad.

The coated zone 12 forms a boundary in the front third delimiting an inner front zone 14 comprising or consisting of a coated free topsheet area. The inner front zone 14 extends along the longitudinal centreline Lc.

The coated zone 12 is furthermore arranged along the first and the second longitudinal side edges 5,6 in the central third 11 of the sanitary pad 1. The coated zone 12 is arranged at a distance from the first and second longitudinal side edges 5,6 in the central third 11 of the sanitary pad 1 and forms a boundary in the central third 11 delimiting, on a respective lateral side thereof, an inner central zone 15 comprising or consisting of a coated free topsheet area.

The distance d from the coated zone 12 to the rear edge 8 may be within the range of from 1 mm to 18 mm, the distance d from the coated zone 12 to the rear edge 8 may be within the range of from 1 mm to 18 mm or from 1 mm to 15 mm. The distance d from the coated zone 12 to the first and the second longitudinal edges 5,6 may be within the range of from 1 mm to 18 mm. This is both an advantage during production, when cutting the final sanitary pads and prevents soiling of the undergarment if the edges are folded during use.

The sanitary pad 1 in Fig. 2 has a length I within the range of from 100 to 300 mm. This is thus a smaller sized type of sanitary pad, such as a pantyliner. The maximum width Wm of the sanitary pad 1 is within the range of from 35 to 110 mm. For smaller sized sanitary pads, such as pantyliner, the risk of folding of the edges may be increased since the edges are closer to the crotch region which may imply increased mechanical forces acting on the edge regions of the sanitary pad, such as for example upon movement of the legs, when sitting down and when changing position.

The amount of formulation in the coated zone is within the range of from 0.2 gsm to 12 gsm, or 0.5 gsm to 7 gsm or 0.5 gsm to 3 gsm. Such amount has been found particularly suitable for the intended purpose.

The formulation may be provided continuously in the coated zone 12. However, the formulation may also be provided discontinuously, for example in stripes, dots or a combination thereof.

In Fig. 2, the inner front zone 14, the inner rear zone 13 and the inner central zone 15 form a coated free zone, being defined by an inner contour of the coated zone 12. The coated free zone forms a head region 18, a body region 19 and a neck region 20, the neck region 20 forming a transitional region between the head region 18 and the body region 19. The head region 18 is positioned in the front half of the sanitary pad 1 and the body region 19 is positioned in the front half and in the rear half of the sanitary pad 1. A transverse width of the coated free zone at the neck region is smaller than a maximum transverse width of the head region.

In Figs. 1-3, the topsheet 2 is printed with printing elements 21 provided in a printed zone 22. The printing elements 21 are printed on the second side 2b of the topsheet 2, i.e., on the opposite side to the side which the coated zone 12 is provided on. The printed zone 22 overlaps with the coated zone 12 and correlates to the coated zone 12, as seen in a depth direction, perpendicular to the longitudinal direction. For example, an outline contour of the printed zone 22 may correspond to an outline contour of the coated zone 12. This may provide a visual cue to the user of where the formulation is located which may provide the user with indication of how to place the article, but also provide the user with signal of the presence of the skin beneficial formulation. In Figs. 1 and 2 there is also an inner coated- and print free zone being free from print and formulation.

The topsheet may include or consist of fibrous nonwoven layer(s) being spunbonded, meltblown, carded, hydroentangled orwetlaid. Suitable nonwoven materials can be composed of natural fibers, such as woodpulp or cotton fibers, synthetic thermoplastic fibers, such as polyolefins, polyesters, polyamides and blends and combinations thereof or from mixtures of natural and synthetic fibers. The materials suited as topshet material should be soft and non-irritating to the skin and be readily penetrated by body fluid, such as menstrual fluid and urine. The formulation may be applied by any suitable method or combination of method, such as for example coating, slot coating, spraying, kiss rolling, dipping or printing.

The backsheet 3 may consist of a thin plastic film, e.g., a polyethylene or polypropylene film, a nonwoven material coated with a liquid impervious material, a hydrophobic nonwoven, all of which resist liquid penetration. Laminates of plastic films and nonwoven materials may also be used. The backsheet material can be breathable to allow vapor to escape from the absorbent structure, while still preventing liquids from passing through the backsheet material.

The absorbent core 4 constitutes the absorbent structure of the article which acquires and stores bodily fluids. The absorbent core may be of any conventional kind. Examples of commonly occurring absorbent materials are cellulosic fluff pulp, tissue, highly absorbent polymers (so called superabsorbents), absorbent foam materials, absorbent nonwoven materials or the like. It is common to combine cellulosic fluff pulp with superabsorbent polymers in an absorbent core. Superabsorbent polymers are water-swellable, water-insoluble organic or inorganic materials capable of absorbing at least about 20 times their own weight of an aqueous solution containing 0.9 weight percent of sodium chloride.

Generally, the core can be of unitary construction, whereby for example the manufacturing process can be simplified. The phrase "unitary construction" in the present context is intended to mean that the absorbent core is constructed from essentially one type of material, this being essentially the same material, or essentially the same combination of two or more materials throughout the absorbent core. Variations in density and concentration of the material may occur, but these are limited to those which may be obtained without incorporation of regions which have been formed separately and then physically joined to each other. For example, when the absorbent core comprises a matrix of hydrophilic fibres and superabsorbent material as described above, the relative concentrations of superabsorbent material and fibres may be different in different parts of the core. However, the absorbent core of unitary construction does not comprise layers or laminates of different composition. Likewise, variations in the density or concentration of various components across the longitudinal direction, the transverse direction or the thickness direction of the absorbent core are acceptable, yet the core should not comprise areas or layers of different composition which are formed separately and later joined together.

A suitable technique for manufacturing the absorbent cores of the present disclosure is mat-forming through an air-laying process. In the process an air-permeable mould is provided. Fibrous material is air-laid into the mould and the mould is filled, whereby an absorbent core is produced with a desired amount of fibrous material.

The sanitary pad 1 may optionally comprise an acquisition layer arranged between the absorbent core 4 and the topsheet 2 and being bonded to the topsheet 2 at least by means of embossings. It may however additionally be adhesively attached to the topsheet. The liquid acquisition layer is adapted to quickly receive and temporarily store discharged liquid before it is absorbed by the absorbent core 4. Such acquisition distribution layers may be composed of for example airlaid nonwoven, spunlace nonwoven or high loft nonwoven or foam materials. The nonwoven material may be hydrophilic. A hydrophilic material may be obtained by adding a surfactant.

An air laid nonwoven can be produced with fluff, wood pulp, and here the fluff fibres are dispersed into a fast-moving air stream and condensed onto a moving screen by means of pressure and vacuum. The web can be bonded with resin and/or thermal plastic resin dispersed within the pulp. The web can be thermobonded (by heat), latex bonded (with adhesive) or multibonded (a combination of thermo and latex bonding) or mechanically bonded (high compression and temperature, bonding by hydrogen). The basis weight of the airlaid nonwoven can suitably be from 35 to 100 gsm.

A high loft material is a nonwoven material and may be substantially free from absorbing fibres and superabsorbent material. The high loft nonwoven material may comprise thermoplastic polymer fibres, and may be selected from but not limited to, polyesters, polyamides and polyolefins such as polyethylenes (PE) and polypropylenes (PP), and may be a mixture of any of these. The high loft material refers to low-density bulky fabrics, as compared to flat, paper-like fabrics. High loft webs are characterised by a relatively low density. This means that there is a relatively high amount of void space between the fibres.

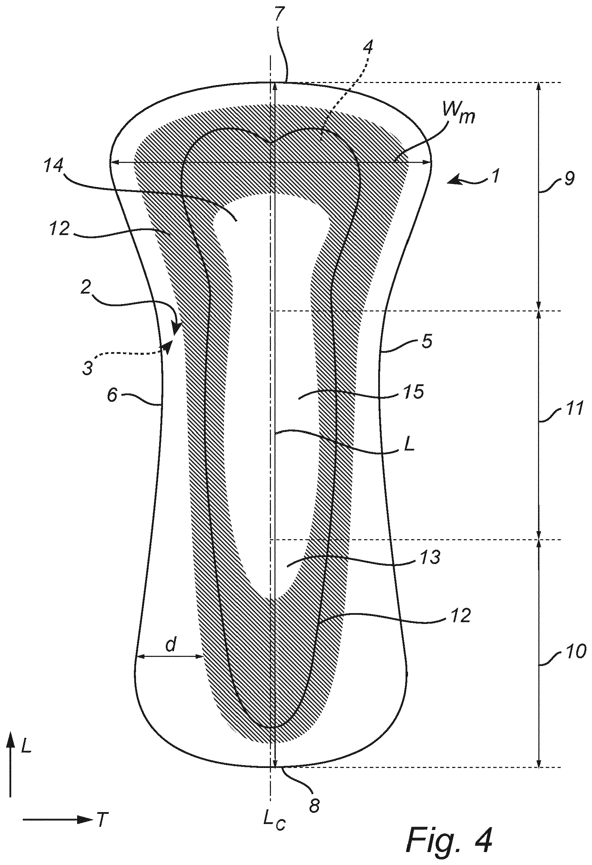

Fig. 4 illustrates a sanitary pad 1 according to the present disclosure. The sanitary pad 1 comprises a topsheet 2, a backsheet 3 and an absorbent core 4 position between the topsheet 2 and the backsheet 3. The sanitary pad 1 extends in a longitudinal direction L and a transverse direction T and has a first longitudinal side edge 5, a second longitudinal side edges 6, a front edge 7 and a rear edge 8. The sanitary pad 1 has a length I extending in the longitudinal direction L and being measured along a longitudinal centreline Lc of the sanitary pad 1 and a maximum width Wm, as measured in the transverse direction T.

The sanitary pad 1 is made up of a front third 9, a rear third 10 and a central third 11 arranged between the front third 9 and the rear third 10, as seen in the longitudinal direction L. The topsheet 2 is provided with a water-soluble formulation , the water-soluble formulation being applied in a coated zone 12 arranged along the rear edge 8 and the first and the second longitudinal side edges 5,6 in the rear third 11 of the sanitary pad 1. The coated zone 12 forms a boundary in the rear third delimiting an inner rear zone 13 comprising or consisting of a coated free topsheet area. The coated zone 12 is arranged at a distance d from the rear edge 8 and from the first and second longitudinal side edges 5,6.

The coated zone 12 furthermore forms a boundary in the front third 9 delimiting an inner front zone 14, extending along the longitudinal centreline Lc and comprising or consisting of a coated free topsheet area, and in the central third 11 of the sanitary pad 1 delimiting an inner central zone 15 comprising or consisting of a coated free topsheet area, third 11 delimiting, on a respective lateral side thereof, an inner central zone 15

The distance d from the coated zone 12 to each of the front and rear edge 7,8, the first and the second longitudinal edges 5,6 is 2 mm or more, optionally within the range of from 1 mm to 18 mm. This is both an advantage during production, when cutting the final sanitary pads and prevents soiling of the undergarment if the edges are folded during use. In the sanitary pad 1 depicted in figure 4 the distance d between the first and second longitudinal side edges 5,6 and the coated zone 12 is greater than in front third 9 and in the central third 11 of the sanitary pad 1.

## Claims

1. A sanitary pad (1), such as a pantyliner, comprising a fluid permeable topsheet (2) and a backsheet (3), the sanitary pad (1) extending in a longitudinal direction (L) and a transverse direction (T) and having first and second longitudinal side edges (5,6) and a front and a rear edge (7.8), the sanitary pad (1) having a length (I) extending in the longitudinal direction (L) and being measured along a longitudinal centreline (Lc) of the sanitary pad (1) and a maximum width (Wm), as measured in the transverse direction (T), the sanitary pad (1) being made up of a front third (9), a rear third (10) and a central third (11) arranged between the front and the rear third (9,10), as seen in the longitudinal direction (L), the topsheet (2) being provided with a water-soluble formulation **characterized in that** the water-soluble formulation is applied in a coated zone (12) arranged along the rear edge (8) and the first and the second longitudinal side edges (5,6) in the rear third (10) of the sanitary pad (1) forming a boundary in the rear third (10) delimiting an inner rear zone (13) comprising or consisting of a coated free topsheet area, the inner rear zone (13) extending along the longitudinal centreline (Lc), the coated zone (12) being arranged at a distance from the rear edge (8) and from the first and second longitudinal side edges (5,6) and covering from 25%, optionally from 35% or from 50% of a total surface area of the topsheet (2) in the rear third (10) of the sanitary pad (1).

2. The sanitary pad (1) according to claim 1, wherein the distance from the coated zone (12) to each of the rear edge (8), the first and the second longitudinal edges (5,6) is 1 mm or more, optionally within the range of from 1 mm to 18 mm.

3. The sanitary pad (1) according to claim 1 or 2, wherein the coated zone (12) covers within the range of from 25 to 80% of the total surface area of the topsheet (2) in the rear third of the sanitary pad (1), optionally the coated zone (12) covers within the range of from 35 to 75% of the total area of the topsheet (2) in the rear third of the sanitary pad (1).

4. The sanitary pad (1) according to any of claims 1 to 3, wherein the coated zone (12) also is arranged along the front edge (7), the first and the second longitudinal side edges (5,6) in the front third (9) of the sanitary pad (1), the coated zone (12) being arranged at a distance from the front edge (7), the first and second longitudinal side edges (5,6) in the front third (9) of the sanitary pad (1), wherein, preferably, the distance from the coated zone (12) to each of the front edge (7), the first and the second longitudinal edges (5,6) is 1 mm or more, optionally within the range of 1 to 18 mm.

5. The sanitary pad (1) according to claim 4, wherein the coated zone (12) forms a boundary in the front third (9) delimiting an inner front zone (14) comprising or consisting of a coated free topsheet area, the inner front zone (14) extending along the longitudinal centreline (Lc), optionally the coated zone (12) covering from 25%, optionally from 35% or from 50% of a total surface area of the topsheet (2) in the front third (10) of the sanitary pad (1).

6. The sanitary pad (1) according to any of claims 1-5, wherein the coated zone is arranged along the first and the second longitudinal side edges in the central third of the sanitary pad, the coated zone being arranged at a distance from the first and second longitudinal side edges in the central third and forms a boundary in the central third delimiting an inner central zone comprising an inner central coated free zone extending along the longitudinal centreline, optionally the inner central coated free zone covers at least 40% of the total area of the topsheet in the central third of the sanitary pad.

7. The sanitary pad (1) according to any one of the preceding claims, wherein the length (I) of the sanitary pad (1) is within the range of from 100 to 300 mm, or 100 to 260 mm.

8. The sanitary pad (1) according to any one of the preceding claims, wherein the maximum width (Wm) of the sanitary pad (1) is within the range of from 35 mm to 110 mm.

9. The sanitary pad (1) according to any one of the preceding claims, wherein the amount of formulation in the coated zone (12) is within the range of from 0.2 gsm to 12 gsm, or 0.5 gsm to 7 gsm or 0.5 gsm to 3 gsm.

10. The sanitary pad (1) according to any one of the preceding claims, wherein the formulation is provided continuously or discontinuously in the coated zone (12).

11. The sanitary pad (1) according to any one of the preceding claims, wherein the coated free zone is defined by an inner contour of the coated zone (12), forms a head region (18), a body region (19) and a neck region (20), the neck region (20) forming a transitional region between the head region (18) and the body region (19), the head region (18) being positioned in the front half of the sanitary pad (1) and the body region (19) being positioned in the front half and in the rear half of the sanitary pad (1), wherein a transverse width of the coated free zone at the neck region (20) is smaller than a maximum transverse width of the head region (18).

12. The sanitary pad (1) according to any one of the preceding claims, wherein the sanitary pad (1) comprises an absorbent core (4) arranged between the topsheet (2) and the backsheet (3), wherein, preferably, the absorbent core (4) has a generally uniform no-load thickness over the total absorbent core.

13. The sanitary pad (1) according to claim 12, wherein the absorbent core (4) constitutes of one absorbent layer.

14. The sanitary pad (1) according to claim 12, wherein the absorbent core (4) comprises two or more absorbent layers, and wherein each of the layers of the absorbent core has the same extension, as seen in the longitudinal direction (L) and the transverse direction (T).

15. The sanitary pad (1) according to any one of the preceding claims, wherein the topsheet comprises a second side being an opposition side to the first side, the second side of the topsheet is printed in a printed zone and the coated zone on the first side of the topsheet partly or completely overlap with the printed zone, as seen in a depth direction perpendicular to the longitudinal direction.

## Patentansprüche

1. Hygienebinde (1), wie beispielsweise eine Slipeinlage, umfassend eine fluiddurchlässige Oberschicht (2) und eine Unterschicht (3), wobei sich die Hygienebinde (1) in einer Längsrichtung (L) und einer Querrichtung (T) erstreckt und erste und zweite Längsseitenkanten (5, 6) sowie eine vordere und eine hintere Kante (7.8) aufweist, wobei die Hygienebinde (1) eine Länge (I), die sich in der Längsrichtung (L) erstreckt und entlang einer Längsmittellinie (Lc) der Hygienebinde (1) gemessen wird, und eine maximale Breite (Wm) aufweist, gemessen in der Querrichtung (T), wobei die Hygienebinde (1) aus einem vorderen Drittel (9), einem hinteren Drittel (10) und einem mittleren Drittel (11) besteht, das zwischen dem vorderen und dem hinteren Drittel (9, 10) angeordnet ist, in Längsrichtung (L) gesehen, wobei die Oberschicht (2) mit einer wasserlöslichen Formulierung versehen ist, **dadurch gekennzeichnet, dass** die wasserlösliche Formulierung in einer Beschichtungszone (12) aufgebracht ist, die entlang der hinteren Kante (8) angeordnet ist, und die ersten und zweiten Längsseitenkanten (5, 6) in dem hinteren Drittel (10) der Hygienebinde (1) eine Grenze in dem hinteren Drittel (10) bilden, die eine innere hintere Zone (13) begrenzt, die einen beschichteten freien Oberschichtbereich umfasst oder aus diesem besteht, wobei sich die innere hintere Zone (13) entlang der Längsmittellinie (Lc) erstreckt, wobei die Beschichtungszone (12) in einem Abstand von der hinteren Kante (8) und von der ersten und zweiten Längsseitenkante (5, 6) angeordnet ist und 25 %, optional 35 % oder 50 % einer Gesamtfläche der Oberschicht (2) in dem hinteren Drittel (10) der Hygienebinde (1) bedeckt.

2. Hygienebinde (1) nach Anspruch 1, wobei der Abstand der Beschichtungszone (12) zu jeder von der hinteren Kante (8), der ersten und der zweiten Längskante (5, 6) 1 mm oder mehr ist, optional im Bereich von 1 mm bis 18 mm liegt.

3. Hygienebinde (1) nach Anspruch 1 oder 2, wobei die Beschichtungszone (12) im Bereich von 25 bis 80 % der Gesamtfläche der Oberschicht (2) in dem hinteren Drittel der Hygienebinde (1) bedeckt, optional die Beschichtungszone (12) im Bereich von 35 bis 75 % der Gesamtfläche der Oberschicht (2) in dem hinteren Drittel der Hygienebinde (1) bedeckt.

4. Hygienebinde (1) nach einem der Ansprüche 1 bis 3, wobei die Beschichtungszone (12) auch entlang der vorderen Kante (7), der ersten und der zweiten Längsseitenkante (5, 6) in dem vorderen Drittel (9) der Hygienebinde (1) angeordnet ist, wobei die Beschichtungszone (12) in einem Abstand von der vorderen Kante (7), der ersten und zweiten Längsseitenkanten (5, 6) in dem vorderen Drittel (9) der Hygienebinde (1) angeordnet ist, wobei der Abstand der Beschichtungszone (12) zu jeder von der vorderen Kante (7), der ersten und der zweiten Längskante (5, 6) vorzugsweise 1 mm oder mehr ist, optional im Bereich von 1 bis 18 mm liegt.

5. Hygienebinde (1) nach Anspruch 4, wobei die Beschichtungszone (12) in dem vorderen Drittel (9) eine Grenze bildet, die eine innere vordere Zone (14) begrenzt, die einen beschichteten freien Oberschichtbereich umfasst oder aus diesem besteht, wobei sich die innere vordere Zone (14) entlang der Längsmittellinie (Lc) erstreckt, wobei die Beschichtungszone (12) optional 25 %, optional 35 % oder 50 % einer Gesamtfläche der Oberschicht (2) in dem vorderen Drittel (10) der Hygienebinde (1) bedeckt.

6. Hygienebinde (1) nach einem der Ansprüche 1-5, wobei die Beschichtungszone entlang der ersten und der zweiten Längsseitenkante in dem mittleren Drittel der Hygienebinde angeordnet ist, wobei die Beschichtungszone in einem Abstand von der ersten und der zweiten Längsseitenkante in dem mittleren Drittel angeordnet ist und eine Grenze in dem mittleren Drittel bildet, die eine innere mittlere Zone begrenzt, die eine innere mittlere beschichtete freie Zone umfasst, die sich entlang der Längsmittellinie erstreckt, wobei die innere mittlere beschichtete freie Zone optional mindestens 40 % der Gesamtfläche der Oberschicht in dem mittleren Drittel der Hygienebinde bedeckt.

7. Hygienebinde (1) nach einem der vorstehenden Ansprüche, wobei die Länge (I) der Hygienebinde (1) im Bereich von 100 bis 300 mm oder 100 bis 260 mm liegt.

8. Hygienebinde (1) nach einem der vorstehenden Ansprüche, wobei die maximale Breite (Wm) der Hygienebinde (1) im Bereich von 35 mm bis 110 mm liegt.

9. Hygienebinde (1) nach einem der vorstehenden Ansprüche, wobei die Menge der Formulierung in der Beschichtungszone (12) im Bereich von 0,2 g/m² bis 12 g/m², oder 0,5 g/m² bis 7 g/m² oder 0,5 g/m² bis 3 g/m² liegt.

10. Hygienebinde (1) nach einem der vorstehenden Ansprüche, wobei die Formulierung in der Beschichtungszone (12) kontinuierlich oder diskontinuierlich vorgesehen ist.

11. Hygienebinde (1) nach einem der vorstehenden Ansprüche, wobei die beschichtete freie Zone durch eine Innenkontur der Beschichtungszone (12) definiert ist, einen Kopfbereich (18), einen Körperbereich (19) und einen Halsbereich (20) bildet, wobei der Halsbereich (20) einen Übergangsbereich zwischen dem Kopfbereich (18) und dem Körperbereich (19) bildet, wobei der Kopfbereich (18) in der vorderen Hälfte der Hygienebinde (1) positioniert ist und der Körperbereich (19) in der vorderen Hälfte und in der hinteren Hälfte der Hygienebinde (1) positioniert ist, wobei eine Querbreite der beschichteten freien Zone in dem Halsbereich (20) kleiner ist als eine maximale Querbreite des Kopfbereichs (18).

12. Hygienebinde (1) nach einem der vorstehenden Ansprüche, wobei die Hygienebinde (1) einen zwischen der Oberschicht (2) und der Unterschicht (3) angeordneten absorbierenden Kern (4) umfasst, wobei der absorbierende Kern (4) vorzugsweise eine im Wesentlichen gleichmäßige Dicke ohne Beladung über den gesamten absorbierenden Kern aufweist.

13. Hygienebinde (1) nach Anspruch 12, wobei der absorbierende Kern (4) aus einer absorbierenden Schicht besteht.

14. Hygienebinde (1) nach Anspruch 12, wobei der absorbierende Kern (4) zwei oder mehr absorbierende Schichten umfasst, und wobei jede der Schichten des absorbierenden Kerns in Längsrichtung (L) und Querrichtung (T) gesehen die gleiche Ausdehnung aufweist.

15. Hygienebinde (1) nach einem der vorstehenden Ansprüche, wobei die Oberschicht eine zweite Seite umfasst, die der ersten Seite gegenüberliegt, wobei die zweite Seite der Oberschicht in einer bedruckten Zone bedruckt ist und die Beschichtungszone auf der ersten Seite der Oberschicht die bedruckte Zone in einer zu der Längsrichtung senkrechten Tiefenrichtung teilweise oder vollständig überlappt.

## Revendications

1. Une serviette hygiénique (1), comme un protège-slip, comprenant une couche supérieure perméable aux fluides (2) et une couche arrière (3), la serviette hygiénique (1) s'étendant dans une direction longitudinale (L) et une direction transversale (T) et ayant des premier et deuxième bords latéraux longitudinaux (5, 6) ainsi qu'un bord avant et arrière (7, 8), la serviette hygiénique (1) ayant une longueur (1) s'étendant dans la direction longitudinale (L) et mesurée le long d'une ligne centrale longitudinale (Lc) de la serviette hygiénique (1) et une largeur maximale (Wm), telle que mesurée dans la direction transversale (T), la serviette hygiénique (1) étant composée d'un tiers avant (9), d'un tiers arrière (10) et d'un tiers central (11) disposé entre les tiers avant et arrière (9, 10), comme on le voit dans la direction longitudinale (L), la couche supérieure (2) étant munie d'une formulation soluble dans l'eau **caractérisée en** ce la formulation soluble dans l'eau est appliquée dans une zone revêtue (12) disposée le long du bord arrière (8) et des premier et deuxième bords latéraux longitudinaux (5, 6) dans le tiers arrière (10) de la serviette hygiénique (1) formant une frontière dans le tiers arrière (10) délimitant une zone arrière intérieure (13) comprenant ou constituée d'une zone de couche supérieure libre de revêtement, la zone arrière intérieure (13) s'étendant le long de la ligne centrale longitudinale (Lc), la zone revêtue (12) étant disposée à distance du bord arrière (8) et des premier et deuxième bords latéraux longitudinaux (5, 6), couvrant à partir de 25 %, éventuellement de 35 % ou de 50 % de la surface totale de la couche supérieure (2) dans le tiers arrière (10) de la serviette hygiénique (1).

2. La serviette hygiénique (1) selon la revendication 1, où la distance entre la zone revêtue (12) et chacun du bord arrière (8), du premier et du second bords longitudinaux (5, 6), est de 1 mm ou plus, éventuellement dans la plage de 1 mm à 18 mm.

3. La serviette hygiénique (1) selon la revendication 1 ou 2, dans laquelle la zone revêtue (12) couvre dans une plage de 25 à 80 % de la surface totale de la couche supérieure (2) dans le tiers arrière de la serviette hygiénique (1), en option la zone revêtue (12) couvre une plage allant de 35 à 75 % de la surface totale de la couche supérieure (2) dans le tiers arrière de la serviette hygiénique (1).

4. La serviette hygiénique (1) selon l'une des revendications 1 à 3, où la zone revêtue (12) est également disposée le long du bord avant (7), des premier et deuxième bords latéraux longitudinaux (5, 6) dans le tiers avant (9) de la serviette hygiénique (1), la zone revêtue (12) étant disposée à distance du bord avant (7), les premier et deuxième bords latéraux longitudinaux (5, 6) dans le tiers avant (9) de la serviette hygiénique (1), où, de préférence, la distance entre la zone revêtue (12) et chacun des bord avant (7), des premier et deuxième bords longitudinaux (5,6) est de 1 mm ou plus, éventuellement dans la plage de 1 à 18 mm.

5. La serviette hygiénique (1) selon la revendication 4, où la zone revêtue (12) forme une frontière dans le tiers avant (9) délimitant une zone avant intérieure (14) comprenant ou constituée d'une zone de couche supérieure libre de revêtement, la zone avant intérieure (14) s'étendant le long de la ligne centrale longitudinale (Lc), optionnellement la zone revêtue (12) couvrant 25 %, éventuellement 35 % ou 50 % de la surface totale de la couche supérieure (2) dans le tiers avant (10) de la serviette hygiénique (1).

6. La serviette hygiénique (1) selon l'une des revendications 1 à 5, où la zone revêtue est disposée le long des premier et deuxième bords latéraux longitudinaux dans le tiers central de la serviette hygiénique, la zone revêtue étant disposée à distance des premier et deuxième bords latéraux longitudinaux dans le tiers central et forme une frontière dans le tiers central délimitant une zone centrale intérieure comprenant une zone centrale intérieure libre de revêtement s'étendant le long de la ligne centrale longitudinale, en option la zone centrale intérieure libre de revêtement couvre au moins 40 % de la surface totale de la couche supérieure dans le tiers central de la serviette hygiénique.

7. La serviette hygiénique (1) selon l'une des revendications précédentes, où la longueur (1) de la serviette hygiénique (1) est comprise entre 100 et 300 mm, ou 100 à 260 mm.

8. La serviette hygiénique (1) selon l'une des revendications précédentes, où la largeur maximale (Wm) de la serviette hygiénique (1) est comprise entre 35 mm et 110 mm.

9. La serviette hygiénique (1) selon l'une des revendications précédentes, où la quantité de formulation dans la zone revêtue (12) se situe dans la plage de de 0,2 gsm à 12 gsm, ou 0,5 gsm à 7 gsm ou 0,5 gsm à 3 gsm.

10. La serviette hygiénique (1) selon l'une des revendications précédentes, où la formulation est fournie de façon continue ou discontinue dans la zone revêtue (12).

11. La serviette hygiénique (1) selon l'une des revendications précédentes, où la zone libre de revêtement est définie par un contour intérieur de la zone revêtue (12), forme une région de tête (18), une région de corps (19) et une région de col (20), la région de col (20) formant une région de transition entre la région de tête (18) et la région de corps (19), la région de tête (18) est positionnée dans la moitié avant de la serviette hygiénique (1) et la région de corps (19) est positionnée dans la moitié avant et dans la moitié arrière de la serviette hygiénique (1), où une largeur transversale de la zone libre de revêtement à la région de col (20) est inférieure à la largeur transversale maximale de la région de tête (18).

12. La serviette hygiénique (1) selon l'une des revendications précédentes, où la serviette hygiénique (1) comprend un noyau absorbant (4) disposé entre la couche supérieure (2) et la couche arrière (3), où, de préférence, le noyau absorbant (4) a une épaisseur sans charge généralement uniforme sur l'ensemble du noyau absorbant.

13. La serviette hygiénique (1) selon la revendication 12, dans laquelle le noyau absorbant (4) est constitué d'une seule couche absorbante.

14. La serviette hygiénique (1) selon la revendication 12, où le noyau absorbant (4) comprend deux couches absorbantes ou plus, et où chacune des couches du noyau absorbant a la même extension, comme on le voit dans la direction longitudinale (L) et la direction transversale (T).

15. La serviette hygiénique (1) selon l'une des revendications précédentes, où la couche supérieure comprend un second côté opposé au premier côté, le second côté de la couche supérieure est imprimée dans une zone imprimée et la zone revêtue sur le premier côté de la couche supérieure chevauche partiellement ou totalement la zone imprimée, comme vu dans une direction de profondeur perpendiculaire à la direction longitudinale.
